# EUROPEAN PATENT SPECIFICATION

(11) **EP 0 599 913 B1**
(45) Date of publication and mention of the grant of the patent: **02.12.1998**
(21) Application number: 92917270.8
(22) Date of filing: 21.08.1992
(51) Int. Cl.: C12P 21/08, C12N 5/20, G01N 33/53

(54) **MONOCLONAL ANTIBODIES TO PUTATIVE HCV NS5 PROTEINS AND METHOS FOR USING SAME**
MONOKLONALE ANTIKÖRPER GEGEN MUTMASSLICHE HEPATITIS C-VIRUS NS5-PROTEINE UND IHRE ANWENDUNGSMETHODEN
ANTICORPS MONOCLONAUX SE FIXANT SUR LES PROTEINES NS5 PUTATIVES DU VIRUS DE L'HEPATITE C, ET PROCEDES D'UTILISATION DE CES ANTICORPS

(30) Priority: 21.08.1991 US 748563
(43) Date of publication of application: 08.06.1994
(73) Proprietor: ABBOTT LABORATORIES, Abbott Park, Illinois 60064-3500 (US)
(72) Inventor: MEHTA, Smriti, U., Libertyville, IL 60048 (US); JOHNSON, Jill, E., Waukegan, IL 60085 (US); DAILEY, Stephen, H., Vernon Hills, IL 60061 (US); DESAI, Suresh, M., Libertyville, IL 60048 (US); DEVARE, Sushil, G., Northbrook, IL 60062 (US)
(74) Representative: Modiano, Guido, Dr.-Ing.
(86) International application number: US9206965
(87) International publication number: WO9304084

(56) References cited:
- EP-A- 0 445 423
- EP-A- 0 461 462
- EP-A- 0 464 287
- EP-A- 0 468 527
- EP-A- 0 472 207
- WO-A-89/04669
- WO-A-90/11089
- 42ND ANNUAL MEETING OF THE AMERICAN ASSOCIATION FOR THE STUDY OF LIVER DISEASES, 2 November 1991, CHICAGO,ILL.,USA TIBBS ET AL 'DETECTION OF ANTIBODIES TO CORE,ENVELOPE,NS-1,NS-3 AND NS-5 REGIONS OF HEPATITIS C VIRUS IN PATIENTS WITH CHRONIC VIRAL HEPATITIS' & HEPATOLOGY, vol.14, no.4(2), 1991 page 68A

## Description

### Background of the Invention

This invention relates generally to antibodies which specifically bind to hepatitis C virus (HCV), and more specifically, relates to a panel of novel hybridoma cells lines which secrete monoclonal antibodies which specifically bind to the putative HCV protein NS5, and methods for using these monoclonal antibodies.

Descriptions of hepatitis diseases causing jaundice and icterus have been known to man since antiquity. Viral hepatitis is now known to include a group of viral agents with distinctive viral organization protein structure and mode of replication, causing hepatitis with different degrees of severity of hepatic damage through different routes of transmission. Acute viral hepatitis is clinically diagnosed by well-defined patient symptoms including jaundice, hepatic tenderness and an elevated level of liver transaminases such as aspartate transaminase and alanine transaminase.

Serological assays currently are employed to further distinguish between hepatitis-A and hepatitis-B. Non-A non-B Hepatitis (NANBH) is a term first used in 1975 that described cases of post-transfusion hepatitis not caused by either hepatitis A virus or hepatitis B virus. Feinstone et al., New Engl. J. Med. 292:454-457 (1975). The diagnosis of NANBH has been made primarily by means of exclusion on the basis of serological analysis for the presence of hepatitis A and hepatitis B. NANBH is responsible for about 90% of the cases of post-transfusion hepatitis. Hollinger et al. in N. R. Rose et al., eds., Manual of Clinical Immunology, American Society for Microbiology, Washington, D. C., 558-572 (1986).

Attempts to identify the NANBH virus by virtue of genomic similarity to one of the known hepatitis viruses have failed thus far, suggesting that NANBH virus has a distinctive genomic organization and structure. Fowler et al., J. Med. Virol. 12:205-213(1983), and Weiner et al., J. Med, Virol. 21:239-247 (1987). Progress in developing assays to detect antibodies specific for NANBH has been hampered by difficulties encountered in identifying antigens associated with the virus. Wards et al., U. S. Patent No. 4,870,076; Wards et al., Proc. Natl. Acad. Sci. 83:6608-6612 (1986); Ohori et al., J. Med. Virol. 12:161-178 (1983); Bradley et al., Proc. Natl. Acad. Sci. 84:6277-6281 (1987); Akatsuka et al., J. Med. Virol. 20:43-56 (1986).

In May of 1988, a collaborative effort of Chiron Corporation with the Centers for Disease Control resulted in the identification of a putative NANB agent, hepatitis C virus (HCV). M. Houghton et al. cloned and expressed in E. coli a NANB agent obtained from the infectious plasma of a chimp. Kuo et al., Science 244:359-361 (1989); Choo et al., Science 244:362-364 (1989). cDNA (copy DNA) sequences from HCV were identified which encode antigens that react immunologically with antibodies present in a majority of the patients clinically diagnosed with NANBH. Based on the information available and on the molecular structure of HCV, the genetic makeup of the virus consists of single stranded linear RNA (positive strand) of molecular weight approximately 9.5 kb, and possessing one continuous translational open reading frame. J. A. Cuthbert, Amer. J. Med. Sci. 299:346-355 (1990). It is a small enveloped virus resembling the Flaviviruses. Investigators have made attempts to identify the NANB agent by ultrastructural changes in hepatocytes in infected individuals. H. Gupta, Liver 8:111-115 (1988); D.W. Bradley J. Virol. Methods 10:307-319 (1985). Similar ultrastructural changes in hepatocytes as well as PCR amplified HCV RNA sequences have been detected in NANBH patients as well as in chimps experimentally infected with infectious HCV plasma. T. Shimizu et al., Proc. Natl. Acad. Sci. 87:6441-6444 (1990).

Considerable serological evidence has been found to implicate HCV as the etiological agent for post-transfusion NANBH. H. Alter et al., N. Eng. J. Med. 321:1494-1500 (1989); Estaben et al., The Lancet: Aug. 5:294-296 (1989); C. Van Der Poel et al., The Lancet Aug. 5:297-298 (1989); G. Sbolli, J. Med. Virol, 30:230-232 (1990); M. Makris et al., The Lancet 335:1117-1119 (1990). Although the detection of HCV antibodies eliminates 70 to 80% of NANBH infected blood from the blood supply system, the antibodies apparently are readily detected during the chronic state of the disease, while only 60% of the samples from the acute NANBH stage are HCV antibody positive. H. Alter et al., New Eng. J. Med. 321:1994-1500 (1989). These data clearly indicated the need for the identification of additional HCV proteins for efficient serodiagnosis of HCV infection. Following the cloning and expression of structural protein CORE and 33C, second generation antibody assays have been developed which employ HCV CORE and 33C proteins in addition to C-100 for the detection of antibodies to HCV in NANB patients. Although the second generation assays have significantly increased the sensitivity of detection, the prolonged interval between exposure to HCV and antibody detection, and the lack of adequate information regarding the profile of immune response to various structural and non-structural proteins raises questions regarding the infectious state of the patient in the antibody negative phase during NANBH infection. Therefore, there is a need for the development of assay systems to identify acute infection to HCV and the presence of HCV.

As further background to the present invention, M. Houghton et al. in WO-A-90/11089 disclose HCV peptides, including peptides containing epitopes belonging to the envelope region, useful in HCV diagnostics and vaccines. Monoclonal antibodies to such peptides are disclosed.

EP-A-0 464 287, which is prior art under the terms of Article 54(3) and (4) EPC, generally discloses the HCV NS5 protein and the possibility of raising antibodies to HCV polypeptides and using them as a diagnostic agent.

EP-A-0 468 527, which is prior art under the terms of Article 54(3) and (4) EPC, discloses HCV NS5 polypeptides and the possibility of raising antibodies to HCV polypeptides and using them as a diagnostic agent.

### Summary of the Invention

The present invention provides a panel of highly specific and novel monoclonal antibodies that can be employed for the detection of putative HCV NS5 antigens. The monoclonal antibodies specifically bind to protein sequences derived from the putative HCV NS5 gene. The hybridomas which produce these monoclonal antibodies are identified as follows: hybridoma H12C65 (A.T.C.C. deposit No. HB 10855) and hybridoma H18C141 (A.T.C.C. deposit No. HB 10854).

The specificity of these monoclonal antibodies enables the advantageous identification of HCV antigen in the putative NS5 region, which identification can be useful in differentiation studies as well as in the diagnosis and evaluation of HCV (NANB) infections.

In a preferred assay format, a test sample which may contain HCV antigens is contacted with a solid phase to which a monoclonal anti-HCV NS5 antibody of the invention or a fragment thereof has been bound, to form a mixture. This mixture is incubated for a time and under conditions sufficient for antigen/antibody complexes to form. The so-formed complexes then are contacted with an indicator reagent comprising a monoclonal antibody of the invention or a fragment thereof, specific for the HCV antigen attached to a signal generating compound to form a second mixture. This second mixture is reacted for a time and under conditions sufficient to form antibody/antigen/antibody complexes. The presence of HCV antigen is determined by detecting the measurable signal generated. The amount of HCV present in the test sample, thus the amount of HCV antigen captured on the solid phase, is proportional to the amount of signal generated.

Alternatively, an indicator reagent comprising a monoclonal antibody of the invention, or fragment thereof, specific for HCV NS5 antigen and a signal generating compound is added to a monoclonal anti-HCV antibody of the invention or fragment thereof coated on a solid phase and the test sample, to form a mixture. This mixture is incubated for a time and under conditions sufficient to form antibody/antigen/antibody complexes. The presence and amount of HCV present in the test sample, and thus the amount of HCV antigen captured on the solid phase, is determined by detecting the measurable signal. The amount of HCV present in the test sample is proportional to the amount of signal generated.

In another alternate assay format, one or a combination of more than one monoclonal antibody of the invention can be employed as a competitive probe for the detection of antibodies to HCV NS5 antigen. For example, HCV NS5 antigens, either alone or in combination, can be coated on a solid phase. A test sample suspected of containing antibody to HCV NS5 antigen then is incubated with an indicator reagent comprising a signal generating compound and a monoclonal antibody of the invention for a time and under conditions sufficient to form antigen/antibody complexes of either the test sample and indicator reagent to the solid phase or the indicator reagent to the solid phase. The reduction in binding of the monoclonal antibody to the solid phase can be quantitatively measured. A measurable reduction in the signal compared to the signal generated from a confirmed negative NANBH test sample would indicate the presence of anti-HCV NS5 antibody in the test sample.

In yet another assay format, a test sample is contacted with a solid phase to which HCV NS5 proteins are attached and an indicator reagent comprising a monoclonal antibody of the invention or fragment thereof specific for HCV NS5 attached to a signal generating compound, to form a mixture. The mixture is incubated for a time and under conditions sufficient for antibody/antigen complexes to form. The presence of anti-HCV present in the test sample is determined by detecting the measurable signal generated, and comparing the signal to the measured signal generated from a known negative sample. A measurable reduction of signal of the test sample, compared to the known negative sample's signal, is indicative of the presence of anti-HCV antibodies. Competitive assays for the detection of anti-HCV antibody using antigens free in solution also can be performed.

The presence of HCV NS5 antigen can be detected in a tissue sample by contacting the tissue sample with an indicator reagent comprising a signal generating compound attached to a monoclonal antibody of the invention which specifically binds to HCV NS5 antigen or fragment thereof, to form a mixture. This mixture is incubated for a time and under conditions sufficient for antigen/antibody complex to form. The presence of HCV NS5 antigen present in the tissue sample is determined by detecting the signal generated.

Also provided are kits useful for determining the presence of HCV NS5 antigen or antibody in test sample that include the monoclonal antibodies of the invention.

### Brief Description of the Drawings

FIG. 1 is an illustration of the location of the recombinant HCV proteins on the HCV genome employed either as immunogens for the generation of monoclonal antibodies or for their characterization.

FIGS. 2A and 2B are Western blot analysis illustrating specific binding of monoclonal antibodies H12C65 (FIG. 2A) and H18C141 (FIG. 2B) to NS5.

### Detailed Description of the Invention

The present invention provides novel monoclonal antibodies to the putative HCV NS5 protein, methods for using the monoclonal antibodies, and kits which contain these monoclonal antibodies.

The monoclonal antibodies of the present invention can be employed in various assay systems to determine the presence, if any, of HCV NS5 proteins in a test sample. Fragments of these monoclonal antibodies provided also may be used. For example, in a first assay format, a monoclonal anti-HCV NS5 antibody of the invention or fragment thereof, or a combination of these antibodies, which has been coated on a solid phase, is contacted with a test sample which may contain HCV NS5 proteins, to form a mixture. This mixture is incubated for a time and under conditions sufficient to form antigen/antibody complexes. Then, an indicator reagent comprising a monoclonal antibody of the invention or a fragment thereof, which specifically binds to the HCV NS5 region, or a combination of these antibodies, to which a signal generating compound has been attached, is contacted with the antigen/antibody complexes to form a second mixture. This second mixture then is incubated for a time and under conditions sufficient to form antibody/antigen/antibody complexes. The presence of HCV NS5 antigen present in the test sample and captured on the solid phase, if any, is determined by detecting the measurable signal generated by the signal generating compound. The amount of HCV NS5 antigen present in the test sample is proportional to the signal generated.

Alternatively, a monoclonal anti-HCV NS5 antibody of the invention or fragment thereof, or a combination of these antibodies which is bound to a solid support, the test sample and an indicator reagent comprising a monoclonal antibody of the invention or fragments thereof, which specifically binds to HCV NS5 antigen, or a combination of these antibodies to which a signal generating compound is attached, are contacted to form a mixture. This mixture is incubated for a time and under conditions sufficient to form antibody/antigen/antibody complexes. The presence, if any, of HCV NS5 proteins present in the test sample and captured on the solid phase is determined by detecting the measurable signal generated by the signal generating compound. The amount of HCV proteins present in the test sample is proportional to the signal generated.

In another alternate assay format, one or a combination of one or more monoclonal antibodies of the invention can be employed as a competitive probe for the detection of antibodies to HCV protein. For example, HCV proteins, either alone or in combination, can be coated on a solid phase. A test sample suspected of containing antibody to HCV NS5 antigen then is incubated with an indicator reagent comprising a signal generating compound and at least one monoclonal antibody of the invention for a time and under conditions sufficient to form antigen/antibody complexes of either the test sample and indicator reagent to the solid phase or the indicator reagent to the solid phase. The reduction in binding of the monoclonal antibody to the solid phase can be quantitatively measured. A measurable reduction in the signal compared to the signal generated from a confirmed negative NANBH test sample indicates the presence of anti-HCV NS5 antibody in the test sample.

In yet another detection method, each of the monoclonal antibodies of the present invention can be employed in the detection of HCV antigens in fixed tissue sections, as well as fixed cells by immunohistochemical analysis.

In addition, these monoclonal antibodies can be bound to matrices similar to CNBr-activated Sepharose and used for the affinity purification of specific HCV proteins from cell cultures, or biological tissues such as blood and liver.

The monoclonal antibodies of the invention can also be used for the generation of chimeric antibodies for therapeutic use, or other similar applications.

The monoclonal antibodies or fragments thereof can be provided individually to detect HCV NS5 antigens. Combinations of the monoclonal antibodies (and fragments thereof) provided herein also may be used together as components in a mixture or "cocktail" of at least one anti-HCV NS5 antibody of the invention with antibodies to other HCV regions, each having different binding specificities. Thus, this cocktail can include the monoclonal antibodies of the invention which are directed to HCV NS5 proteins and other monoclonal antibodies to other antigenic determinants of the HCV genome. Examples of other monoclonal antibodies useful for these contemplated cocktails include those to HCV C-100, HCV 33C, HCV CORE, HCV NS1 and/or HCV putative ENV, which are disclosed in, for example, in WO 92/08738 and WO 92/13892.

This cocktail of monoclonal antibodies as described herein would be used in the assay formats detailed herein in place of the monoclonal antibody to NS5, and thus would be able to detect the NS5 and other HCV antigens.

The polyclonal antibody or fragment thereof which can be used in the assay formats should specifically bind to HCV putative NS5 region or other HCV proteins used in the assay, such as HCV C-100 protein, HCV 33C protein, HCV E2/NS1 or HCV CORE protein. The polyclonal antibody used preferably is of mammalian origin; human, goat, rabbit or sheep anti-HCV polyclonal antibody can be used. Most preferably, the polyclonal antibody is rabbit polyclonal anti-HCV antibody. The polyclonal antibodies used in the assays can be used either alone or as a cocktail of polyclonal antibodies. Since the cocktails used in the assay formats are comprised of either monoclonal antibodies or polyclonal anlibodies having different HCV specificity, they would be useful for diagnosis, evaluation and prognosis of HCV infection, as well as for studying HCV protein differentiation and specificity.

Test samples which can be tested by the methods of the present invention described herein include human and animal body fluids such as whole blood, serum, plasma, cerebrospinal fluid, urine, biological fluids such as cell culture supernatants, fixed tissue specimens and fixed cell specimens.

The "solid phase" is not critical and can be selected by one skilled in the art. Thus, latex particles, microparticles, magnetic or non-magnetic beads, membranes, plastic tubes, walls of microtiter wells, glass or silicon chips and sheep red blood cells are all suitable examples. Suitable methods for immobilizing peptides on solid phases include ionic, hydrophobic, covalent interactions and the like. A "solid phase", as used herein, refers to any material which is insoluble, or can be made insoluble by a subsequent reaction. The solid phase can be chosen for its intrinsic ability to attract and immobilize the capture reagent. Alternatively, the solid phase can retain an additional receptor which has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid phase and which has the ability to immobilize the capture reagent through a specific binding reaction. The receptor molecule enables the indirect binding of the capture reagent to a solid phase material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, and other configurations known to those of ordinary skill in the art.

It is contemplated and within the scope of the invention that the solid phase also can comprise any suitable porous material with sufficient porosity to allow access by detection antibodies and a suitable surface affinity to bind antigens. Microporous structures are generally preferred, but materials with gel structure in the hydrated state may be used as well. Such useful solid supports include:
natural polymeric carbohydrates and their synthetically modified, cross-linked or substituted derivatives, such as agar, agarose, cross-linked alginic acid, substituted and cross-linked guar gums, cellulose esters, especially with nitric acid and carboxylic acids, mixed cellulose esters, and cellulose ethers;
natural polymers containing nitrogen, such as proteins and derivatives, including cross-linked or modified gelatins;
natural hydrocarbon polymers, such as latex and rubber;
synthetic polymers which may be prepared with suitably porous structures, such as vinyl polymers, including polyethylene, polypropylene, polystyrene, polyvinylchloride, polyvinylacetate and its partially hydrolyzed derivatives, polyacrylamides, polymethacrylates, copolymers and terpolymers of the above polycondensates, such as polyesters, polyamides, and other polymers, such as polyurethanes or polyepoxides;
porous inorganic materials such as sulfates or carbonates of alkaline earth metals and magnesium, including barium sulfate, calcium sulfate, calcium carbonate, silicates of alkali and alkaline earth metals, aluminum and magnesium; and aluminum or silicon oxides or hydrates, such as clays, alumina, talc, kaolin, zeolite, silica gel, or glass (these materials may be used as filters with the above polymeric materials); and
mixtures or copolymers of the above classes, such as graft copolymers obtained by initializing polymerization of synthetic polymers on a pre-existing natural polymer. All of these materials may be used in suitable shapes, such as films, sheets, or plates, or they may be coated onto or bonded or laminated to appropriate inert carriers, such as paper, glass, plastic films, or fabrics.

The porous structure of nitrocellulose has excellent absorption and adsorption qualities for a wide variety of reagents including monoclonal antibodies. Nylon also possesses similar characteristics and also is suitable.

It is contemplated that such porous solid supports described hereinabove are preferably in the form of sheets of thickness from about 0.01 to 0.5 mm, preferably about 0.1 mm. The pore size may vary within wide limits, and is preferably from about 0.025 to 15 microns, especially from about 0.15 to 15 microns. The surfaces of such supports may be activated by chemical processes which cause covalent linkage of the antigen or antibody to the support. The irreversible binding of the antigen or antibody is obtained, however, in general, by adsorption on the porous material by poorly understood hydrophobic forces. Suitable solid supports also are described in EP-A-0 353 590.

The indicator reagent comprises a signal generating compound (label) which is capable of generating a measurable signal detectable by external means conjugated (attached) to a specific binding member for HCV. "Specific binding member as used herein means a member of a specific binding pair. That is, two different molecules where one of the molecules through chemical or physical means specifically binds to the second molecule. In addition to being an antibody member of a specific binding pair for HCV, the indicator reagent also can be a member of any specific binding pair, including either hapten-anti-hapten systems such as biotin or anti-biotin, avidin or biotin, a carbohydrate or a lectin, a complementary nucleotide sequence, an effector or a receptor molecule, an enzyme cofactor and an enzyme, an enzyme inhibitor or an enzyme, and the like. An immunoreactive specific binding member can be an antibody, an antigen, or an antibody/antigen complex that is capable of binding either to HCV as in a sandwich assay, to the capture reagent as in a competitive assay, or to the ancillary specific binding member as in an indirect assay.

The various signal generating compounds (labels) contemplated include chromogens, catalysts such as enzymes, luminescent compounds such as fluorescein and rhodamine, chemiluminescent compounds such as acridinium, phenanthridinium and dioxetane compounds, radioactive elements, and direct visual labels. Examples of enzymes include alkaline phosphatase, horseradish peroxidase, beta-galactosidase, and the like. The selection of a particular label is not critical, but it will be capable of producing a signal either by itself or in donjunction with one or more additional substances.

Other embodiments which utilize various other solid phases also are contemplated and are within the scope of this invention. For example, ion capture procedures for immobilizing an immobilizable reaction complex with a negatively charged polymer, described in EP-A-0 326 100 and EP-A-0 406 473, can be employed according to the present invention to effect a fast solution-phase immunochemical reaction. An immobilizable immune complex is separated from the rest of the reaction mixture by ionic interactions between the negatively charged poly-anion/immune complex and the previously treated, positively charged porous matrix and detected by using various signal generating systems previously described, including those described in chemiluminescent signal measurements as described in EP-A-0 273 115.

Also, the methods of the present invention can be adapted for use in systems which utilize microparticle technology including in automated and semi-automated systems wherein the solid phase comprises a microparticle. Such systems include those described in EP 0 425 633 and EP 0 424 634.

The use-of scanning probe microscopy (SPM) for immunoassays also is a technology to which the monoclonal antibodies of the present invention are easily adaptable. In scanning probe microscopy, in particular in atomic force microscopy, the capture phase, for example, at least one of the monoclonal antibodies of the invention, is adhered to a solid phase and a scanning probe microscope is utilized to detect antigen/antibody complexes which may be present on the surface of the solid phase. The use of scanning tunnelling microscopy eliminates the need for labels which normally must be utilized in many immunoassay systems to detect antigen/antibody complexes.

The use of SPM to monitor specific binding reactions can occur in many ways. In one embodiment, one member of a specific binding partner (analyte specific substance which is the monoclonal antibody of the invention) is attached to a surface suitable for scanning. The attachment of the analyte specific substance may be by adsorption to a test piece which comprises a solid phase of a plastic or metal surface, following methods known to those of ordinary skill in the art. Or, covalent attachment of a specific binding partner (analyte specific substance) to a test piece which test piece comprises a solid phase of derivatized plastic, metal, silicon, or glass may be utilized. Covalent attachment methods are known to those skilled in the art and include a variety of means to irreversibly link specific binding partners to the test piece. If the test piece.is silicon or glass, the surface must be activated prior to attaching the specific binding partner. Activated silane compounds such as triethoxy amino propyl silane (available from Sigma Chemical Co., St. Louis, MO), triethoxy vinyl silane (Aldrich Chemical Co., Milwaukee, WI), and (3-mercaptopropyl)-trimethoxy silane (Sigma Chemical Co., St. Louis, MO) can be used to introduce reactive groups such as amino-, vinyl, and thiol, respectively. Such activated surfaces can be used to link the binding partner directly (in the cases of amino or thiol) or the activated surface can be further reacted with linkers such as glutaraldehyde, bis (succinimidyl) suberate, SPPD 9 succinimidyl 3-[2-pyridyldithio] propionate), SMCC (succinimidyl-4-[N-maleimidomethyl] cyclohexane-1-carboxylate), SIAB (succinimidyl [4-iodoacetyl] aminobenzoate), and SMPB (succinimidyl 4-[1-maleimidophenyl] butyrate) to separate the binding partner from the surface. The vinyl group can be oxidized to provide a means for covalent attachment. It also can be used as an anchor for the polymerization of various polymers such as poly acrylic acid, which can provide multiple attachment points for specific binding partners. The amino surface can be reacted with oxidized dextrans of various molecular weights to provide hydrophilic linkers of different size and capacity. Examples of oxidizable dextrans include Dextran T-40 (molecular weight 40,000 daltons), Dextran T-110 (molecular weight 110,000 daltons), Dextran T-500 (molecular weight 500,000 daltons), Dextran T-2M (molecular weight 2,000,000 daltons) (all of which are available from Pharmacia, Piscataway, N.J.,U.S.A.), or Ficoll (molecular weight 70,000 daltons (available from Sigma Chemical Co., St. Louis, MO). Also, polyelectrolyte interactions may be used to immobilize a specific binding partner on a surface of a test piece by using techniques and chemistries described by EP-A-0 326 100 and EP-A-0 406 473.

The preferred method of attachment is by covalent means. Following attachment of a specific binding member, the surface may be further treated with materials such as serum, proteins, or other blocking agents to minimize non-specific binding. The surface also may be scanned either at the site of manufacture or point of use to verify its suitability for assay purposes. The scanning process is not anticipated to alter the specific binding properties of the test piece.

While the present invention discloses the preference for the use of solid phases, it is contemplated that the peptides of the present invention can be utilized in non-solid phase assay systems. These assay systems are known to those skilled in the art, and are considered to be within the scope of the present invention.

It is contemplated that the reagent employed for the assay can be provided in the form of a kit with one or more containers such as vials or bottles, with each container containing a separate reagent such as a monoclonal antibody, or a cocktail of monoclonal antibodies, employed in the assay.

The following examples demonstrate the advantages and utility of this invention for serodiagnosis of HCV by describing methods for the development, characterization, epitope mapping and clinical utility of these monoclonal antibodies. The methods used for monoclonal antibody development follow procedures known in the art and detailed on Kohler and Milstein, Nature 256:494 (1975) and reviewed in J.G.R. Hurrel, ed., Monoclonal Hybridoma Antibodies: Techniques and Applications, CRC Press, Inc., Boco Ratan, FL (1982). Another method of monoclonal antibody development which is based on the Kohler and Milstein method is that of L. T. Mimms et al., Virology 176:604-619 (1990).

### EXAMPLES

### EXAMPLE 1

### Immunization of Mice with pHCV45

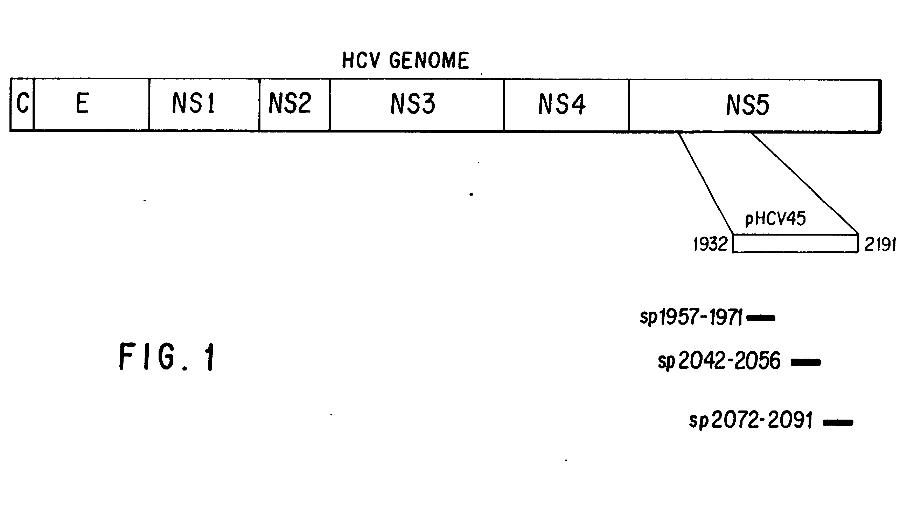

E. coli derived recombinant antigens encoded by HCV sequences, designated as pHCV45, (HCV NS5, a.a. 1932-2191, SEQ. ID. NO. 1) was employed as immunogen for the generation of murine monoclonal antibodies specific for HCV NS5. Eight individual oligonucleotides representing HCV a.a.1932-2191 were ligated together and cloned as a 793 base pair EcoRI-BamHI fragment into the CKS (E. coli CMP-KDO Synthetase) fusion vector pJ0200. Detailed information on the synthesis, cloning and expression of this recombinant protein is disclosed in EP-A-0 472 207. The protein was prepared for immunization with appropriate adjuvants after purification with protein purification methods known to those skilled in the art. FIG. 1 shows the location of recombinant HCV protein pHCV 45 on the genome.

On day one, BALB/c mice received 50 µg of purified pHCV45 in 200 µl of Freund's complete adjuvant injected intraperitoneally (i.p.) at two sites. A second immunization was done after 21 and 35 days with 50 µg of pHCV45 in incomplete Freund's adjuvant. Mice were bled on day 41 and the immune response to pHCV45 was assessed by enzyme linked immunoassay (EIA) and Western blot analysis. Fusion was performed after allowing the mice to rest for at least eight weeks.

### Enzyme-Linked Immunoassay (EIA)

The immune response to the immunizing antigen was assessed by microtiter EIA and Western blot analysis. Wells of microtiter plates were coated with 100 µl of purified recombinant protein (pHCV 45, SEQ. ID. NO. 1) on 0.1M bicarbonate buffer at pH 9.5. After washing with Phosphate Buffered Saline (PBS) which also contained 0.01% sodium dodecyl sulfate (SDS) and 0.05% Tween-20® (available from Bio-Rad Laboratories, Richmond, CA), free sites were overcoated with 1% BSA in bicarbonate buffer at pH 9.5. Plates were stored at 4°C following a final wash. Sera from native or immunized mice were serially diluted in 100 µl of dilution buffer which contained 20 mM sodium phosphate, pH 7.4, 0.15M NaCI, 20% normal goat serum, 10% fetal calf serum, 5 mM EDTA, 10 mM EGTA, 50 mM Tris, 0.2% Tween-20®, with sodium azide as a preservative (at pH 6.8). The diluted sera were reacted with the antigen for three (3) hours at 37°C. The plates were washed and 100 µl of appropriately diluted goat anti-mouse IgG (heavy [h] and light [I] chain) Horseradish Peroxidase (HRPO)-conjugated antibody (Jackson Immunochemicals, West Grove, PA) was added. The plates were incubated at 37°C for two (2) hours. After a final wash, 100 µl of o-phenylenediamine:2 HCL (OPD) color reagent was added. The reaction was carried out at room temperature for 10 to 30 minutes, and then stopped by the addition of 1 ml of 1 N H2S04. The absorbance at 492/600 nm was recorded, which was found to be directly proportional to the amount of specific antibody bound to the respective antigen.

### Western Blot Analysis

Approximately 300 µg of purified rHCV protein (pHCV-45) (SEQ. ID. NO. 1) were treated with SDS and 2-mercaptoethanol at 95°C, and electrophoresed in a 12% polyacrylamide-SDS gel (Laemmli et al., Nature 227:680-685 (1970). Proteins were transferred overnight from the gel to nitrocellulose by electrophoresis at 100 mamp, or transferred in 1-2 hours at 1.0 amp, in a standard transfer buffer which comprised 25 mM Tris [(Hydroxymethyl) Aminomethane] 192 mM glycine, and 2.0% methanol, pH 8.3. (Towbin et al., Proc. Natl. Acad. Sci. 73:4350-4354 [1979]). After transferring the proteins and blocking the nitrocellulose with 5% dry milk in PBS, the nitrocellulose was cut into strips (each strip containing approximately 5 µg of rHCV which then were used to determine the presence of anti-HCV antibody in test sera [or other samples]). Reaction mixtures consisted of a nitrocellulose strip incubated with an appropriate amount of test sample in 2.0 ml of buffer (20 mM Tris, 1 mM EDTA, 0.2 M NaCI, 0.3% Triton X-100® and 2 mg.ml bovine serum albumin (BSA), pH 7.5, 5% E. coli lysate amd 3% CKS lysate overnight at 4°C. The strips were washed with buffered detergent (10 mM phosphate buffered saline (PBS) pH 7.5, containing 0.1% SDS and 0.5% Triton X-100®), followed by addition of goat anti-mouse IgG antibody conjugated to HRPO. The strips were incubated for one to two hours at room temperature, followed by washing with buffered. detergent. Finally, antibody bound to the protein was visualized by addition of freshly prepared HRP color reagent (Bio-Rad Laboratories, Richmond CA) (120 mg dissolved in 40 ml ice-cold methanol, then diluted into 200 ml Tris buffered saline [TBS] pH 7.8, containing 120 µl of 30% hydrogen peroxide. This assay demonstrated the presence of antibody to the recombinant protein with which the mice had been immunized.

### EXAMPLE 2

### Cell Fusion

Upon demonstration of specific anti-HCV antibody present at reasonable titers in sera of immunized mice, the mice were allowed to rest for at least eight weeks prior to a pre-fusion boost of antigen. The pre-fusion antigen boost then was performed by intravenous (IV) tail vein injection of approximately 40 µg of respective purified HCV recombinant protein. Three days later the mice were sacrificed, and their spleens which contained anti-HCV antibody-producing cells were disrupted into single cells. These single cell suspensions were treated with 0.83% NH₄CI to remove red blood cells, and then these suspensions were mixed with SP2/0 cells at a 10:1 (SP2/0:spleen cells) ratio. The mixed cells were centrifuged, washed once with serum-free medium, and again centrifuged. The fusogen polyethylene glycol (PEG) was used to form hybrids of the immune donor spleen cells with the myeloma cell line SP2/0 (HRPT neg.). Kohler and Milstein, Nature 356:494 (1975), and reviewed in J.G.R. Hurrel, ed., Monoclonal Hybridoma Antibodies: Techniques and Applications, CRC Press, Inc., Boco Ratan, FL (1982). Briefly, fusion of the spleen and SP2/0 cells was accomplished by exposing the pellet to 40% PEG (ATCC, mw 1300-1600) In serum-free Iscoe's Modified Dulbecco's Medium (IMDM) for two minutes. The PEG and cell suspension was diluted slowly by the addition of 20 ml of serum-free IMDM over a period of five minutes, followed by collection of the cells by centrifugation. The supernatant was decanted and replaced with 30 ml IMDM containing 20% fetal bovine serum (FBS) (Hyclone Laboratories, Logan, Utah) with HAT (hypoxanthene, aminopterin and thymidine) media in order to select for hybridomas. Spleen cells from one non-immune BALB/c mouse also were added as a feeder layer. The cells were plated at 0.1 ml/well in three 96-well tissue culture plates. An additional 0.1 ml of HAT media was added to each well three days later. At weekly intervals thereafter, one-half the media was replaced with IMDM containing 20% FBS with HT (hypoxanthene and thymidine), and hybrids were allowed to grow for an additional seven to fourteen days.

It was found that some of the hybrids were composed of spleen cells making antibody to HCV fused with SP2/0 cells. Briefly, the fusogen promoted fusion of spleen cell and SP2/0 cell membranes, which formed a heterokaryon containing nuclei of both cells. Eventually, the dissimilar nuclei fuse produced a single nucleus capable of synchronous mitosis. As the fused cells divided, the hybrid stabilized by losing chromosomes of each nucleus. The fused cells were plated into multiple 96-well plates at 10⁵ to 10⁶ cells per well. The hybrid cells formed from SP2/0:spleen cell fusions were selectively propagated by culturing in HAT medium. All unused SP2/0 or SP2/0:SP2/0 fused cells were prevented from growing aminopterin, and unfused spleen cells on spleen:spleen fused cells died off in culture. Only SP2/0:spleen cell hybrids grew in the HAT selective medium.

### EXAMPLE 3

### Screening and Cloning of Monoclonal Antibodies

After 10 to 14 days, culture fluids from wells containing hybridoma cell growth were screened for the presence of a monospecific antibody as follows. Each of the hybridoma culture fluids was tested on a plate coated with the immunogen as well as on a plate coated with CKS protein (fusion partner used for HCV protein SEQ. ID. NO. 1) by the EIA procedure described in Example 1. Hybridoma culture fluids reacting specifically to the immunogen, i.e., HCV protein, and not the CKS fusion partner were selected for further analysis by Western blot analysis. EIA-positive hybridoma culture fluids were tested for their reactivity to the HCV protein pHCV 45 (SEQ. ID. NO. 1) as well as CKS by Western blot analysis as described in Example 1. Hybrid samples reacting specifically with the HCV protein but not with the CKS protein by both EIA and Western blot were identified, and selected for cloning by the limiting dilution method, using the guidelines outlines by J. W. Goding, Monoclonal Antibodies: Principles and Practices, Academic Press, New York (1983). Culture supernatant of cloned samples were tested again by EIA with the immunogen and the CKS protein as described above in Example 1, for the confirmation of monospecific reactivity to HCV protein sequence SEQ. ID. NO. 1. Clones with strongest reactivity specifically to the protein were selected for expansion and further analysis.

### EXAMPLE 4

### Amplification of Antibody Yields by Ascites Method

In order to obtain greater amounts of monoclonal antibodies, 10 to 20 million cloned cells of the desired hybridoma cell line were inoculated into a BALB/c mouse previously treated i.p. with 0.5 ml pristane (2,6,10,14-tetramethylpentadecane) by the method outlined in J.G.R. Hurrel, ed., Monoclonal Hybridoma Antibodies: Techniques and Applications, CRC Press, Inc., Boco Ratan, FL (1982). Pristane treatment enhanced growth of mouse myeloma hybrids within the peritoneum of the mouse, and the ascites fluids which formed were rich in the monoclonal antibody secreted by the hybrid cells. After formation of adequate ascites fluid (approximately seven days), the mice were sacrificed and the ascites were withdrawn from the peritoneum, clarified by centrifugation and stored at -20°C. Monoclonal antibodies from ascites fluid were purified using protein-A sepharose (according to J.G.R. Hurrell ed., supra). All characterization procedures described herein were performed with either culture supernatants, ascites fluids or protein-A purified IgG.

### EXAMPLE 5

### Characterization of Monoclonal Antibodies

### EIA

Enzyme-linked immunoassay as described in Example 1 was used to determine the specificity of each of the monoclonal antibodies to NS5. Briefly, clarified ascites fluids or protein-A purified IgG were reacted in serial dilutions in microtiter plates coated with either a) the immunogen pHCV45 (SEQ. ID. NO. 1), b) CKS protein (fusion partners used for cloning and expression of the immunogen. Specificity of each of the monoclonal antibodies for the pHCV45 protein was confirmed by specific activity of the monoclonal antibody to the immunogen but not with CKS protein.

### Western Blot Analysis

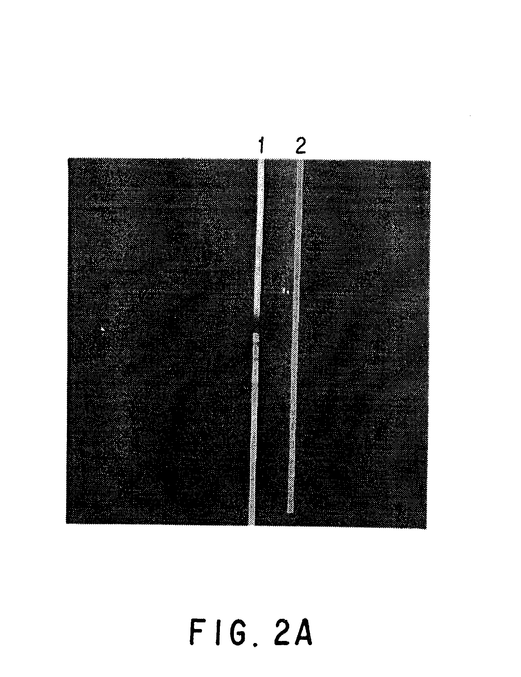

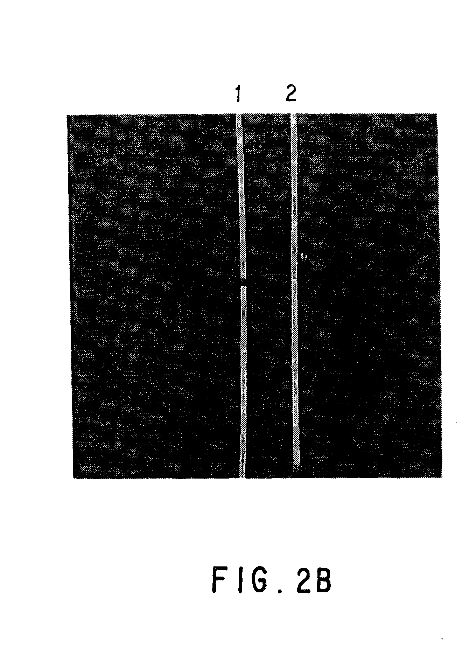

The general protocol for Western blot analysis is as described in Example 1, except that CKS lysate was excluded from the dilution buffer. Briefly, approximately 300 µg of either a) the immunogen pHCV45 or b) CKS protein (fusion partner for the immunogen) were electrophoresed and transferred to the nicrocellulose. After blocking free sites on nitrocellulose, 2 mm side strips were cut. Each of the monoclonal antibodies was tested for reactivity against the immunogen and CKS. Specificity of each of the monoclonal antibodies was confirmed as described for EIA analysis. These data are illustrated in FIG.2. Referring to FIG.2A and 2B, the photographs show mono-specific binding of each monoclonal antibody of the invention to its specific protein. FIG. 2A shows the binding of monoclonal antibody H12C65, wherein lane 1 contains pHCV45 (SEQ.ID.NO.1) and lane 2 contains CKS vector protein only. FIG. 2B shows the binding of monoclonal antibody H18C141, wherein lane 1 contains pHCV45 (SEQ.ID.NO.1) and lane 2 contains CKS vector protein only.

### Isotype

The isotypes of each of the monoclonal antibodies was determined by using an isotyping kit (Amersham, Arlington Heights, IL) and following the instructions included with it. Briefly, the tissue culture supernatant of each monoclonal antibody and appropriate controls were reacted at a 1:5 dilution with strips coated with specific anti-isotype antibody, provided in the kit described above. Assay protocol was followed exactly according to the manufacturer's instructions.

### Competition with Immune Human Sera

In order to establish whether each of the monoclonal antibodies recognized an epitope that is immunologic in humans, a competition assay was performed as follows. Each of the monoclonal antibodies was tested in an assay where the monoclonal antibody competed with a human sera seropositive for antibody to NS5 (SEQ. ID. NO. 1) for the binding to the respective antigen. Briefly, a human serum from an individual infected with NANBH and strongly seropositive for antibodies to NS5 protein of HCV was included in the reaction mixture with each of the monoclonal antibodies at a final concentration of 10%. Microtiter EIA was carried out as described in Example 1. A greater than 50% inhibition in the binding of the monoclonal antibody to the respective protein by the immune human sera was considered as competitive.

### EXAMPLE 6

### Epitope Mapping

Monoclonal antibodies to HCV proteins were mapped to the specific region of the protein by (a) Western blot reactivity of each of the monoclonal antibodies with subfragments of the respective HCV proteins and (b) reactivity with several synthetic peptides selected for respective protein sequences, by microtiter EIA. Specific additional details for mapping are detailed where applicable for an individual monoclonal antibody.

### Reactivity with Synthetic Peptides

Several amino acid sequences were selected from different regions of HCV protein NS5. A list of the peptides used for the epitope mapping of these monoclonal antibodies is listed below in TABLE 1.

**TABLE 1**

| Epitope Mapping with Synthetic Peptides | | | |
|---|---|---|---|
| REGION OF HCV GENOME | MONOCLONAL TESTED | PEPTIDE a.a. | REACTIVITY OF EACH WITH PEPTIDE |
| NS5 | H12C65 | sp 1957-1971* | none |
| | H18C141 | sp 2042-2056** | none |
| | | sp. 2072-2091*** | |

| | | | |
|---|---|---|---|
| * SEQ. ID. NO. 2, | | | |
| **SEQ. ID. NO. 3, and | | | |
| *** SEQ. ID. NO. 4. | | | |

Each of these peptides were assembled on a resin support by a stepwise solid phase synthesis, starting with the carboxy terminal residue. A procedures was employed similar to that described in E. Gross and T. Heinehofer, eds., Barary and Merrifield, The Peptides 2:1284, Academic Press, New York, New York (1980), using a reaction vessel of an Applied Biosystems Synthesizer Model 430A. After cleavage of the peptide from the resin, the peptide was washed with diethyl ether and extracted in 40% acetic acid solution. Crude peptide obtained after lyophilization of the aqueous solution was employed as the antigen target for epitope mapping experiments. Briefly, each of the peptides tested was coated on microtiter wells at a concentration of 10 µg/ml in bicarbonate buffer at pH 9.5. EIA was performed in the manner described in Example 1. Monoclonal antibody showing reactivity four times the negative control was considered positive.

### EXAMPLE 7

### EIA for the Detection of HCV Proteins in Biological Samples Preparation of Rabbit Polyclonal Antibodies Against HCV NS5 Region

Young rabbits (3-4 months old and weighing approximately 2-3 kg) (available from Hazelton Labs, Denver PA) are immunized with 100-150 µg of highly purified HCV NS5 protein as described in Example 1 in Freund's complete adjuvant by intra-muscular (i.m.) injection at four different sites. Subsequently, two immunizations are carried out at two week intervals in similar fashion in Freund's incomplete adjuvant. Immune response of the rabbits is monitored by EIA and Western blot analysis. Rabbits are bled when acceptable immune response to the protein is achieved. IgG from the immune rabbit sera is purified by Protein-A sepharose affinity chromatography, by methods known to those in the art.

### Coating of Solid Phase

Rabbit IgG is prepared as herein described and then is coated on polystyrene beads as the solid support for capture of NS5 antigens in test samples. The polystyrene beads are washed with distilled water and incubated at 40°C for two hours with 5-10 µg/ml of purified HCV NS5 synthetic peptide rabbit IgG in a buffer solution (0.1 M Tris, 0.5 M NaCI, 0.0022% Triton X-100®, pH 8.5). The beads are washed once with PBS and then soaked in 0.1% Triton X-100® in PBS for approximately one hour at 40°C. After washing twice with PBS, the beads are overcoated with 3% bovine serum albumin (BSA) in PBS for approximately one hour at 40°C. Finally, the beads are overcoated with 5% sucrose solution in PBS and dried under nitrogen. Anti-HCV human polyclonal IgG, purified from sera of individuals seropositive for HCV antibodies to NS5 also is coated in similar fashion.

### EIA

Monoclonal antibodies specific for HCV NS5 are screened for use as the probe for detection of HCV proteins in a test sample by EIA. Briefly, each of the monoclonal antibodies is incubated with the NS5 antigen in the presence of polysytrene beads coated with anti-HCV rabbit polyclonal IgG. The protocol for EIA is similar to that described hereinbelow.

200 µl of test specimen suspected of containing antigen to HCV NS5 protein is incubated in a reaction tray with 50 µl of monoclonal antibody of the invention (at a final protein concentration of about 5-10 µg/ml diluted in a buffer containing 20 mM Tris, 0.1 mM NaCl, 1 mM EDTA, 3.0% BSA, 0.3% Tween-20® and 10% FBS at pH 7.5), and a bead coated with HCV rabbit IgG (prepared as described hereinabove). Overnight incubation at ambient room temperature is performed, and then the beads are washed with distilled water and 200 µl of appropriately dilution Horseradish Peroxidase labeled goat anti-mouse IgG (H & L) (Jackson Immunoresearch, West Grove, PA) is added. Incubation with the labeled probe is carried out at about 40°C for approximately two hours. Beads are washed and transferred to reaction tubes containing 300 µl of O-phenylenediamine (OPD) color reagent. The reaction is carried out at ambient room temperature in the dark for about 30 minutes, and then it is stopped by the addition of 1 ml of 1N H₂SO₄. Absorbance is recorded at 492/600 nm. A negative control previously screened and confirmed to be negative for NANBH infection is included in the experiment. The positive control consists of a solution of synthetic peptide to NS5 in the buffer solution described hereinabove. Triplicates of both positive and negative control are included with each set of experiments.

In order to-determine the efficiency of the antigen capture assay for the detection of HCV NS5 in a sample, various concentrations of recombinant HCV NS5 protein, ranging from 100 ng protein/ml to 100 pg protein/ml are diluted in the buffer mentioned hereinabove. The EIA procedure described above is performed with each of the diluted panel members. For the purposes of comparison, each of the panel members is tested with (a) anti-HCV rabbit polyclonal antibody on the solid phase and (b) anti-HCV human polyclonal antibody on the solid phase. The efficiency of the assay then is determined by evaluating data obtained.

The hybridomas which produce the monoclonal antibodies of the invention are identified as hybridoma H12C65 producing monoclonal antibody H12C65, and hybridoma H18C141 producing monoclonal antibody H18C141. Hybridomas H12C65 and H18C141 were deposited at the American Type Culture Collection (ATCC), 12301 Parklawn Drive, Rockville, Maryland 20852 as of August 20, 1991, and have been accorded the following deposit numbers: hybridoma H12C65 was accorded ATCC deposit number HB 10855, and hybridoma H18C141 was accorded ATCC deposit number HB 10854.

Thus, the novel monoclonal antibodies of the invention can be used in a variety of ways. These monoclonal antibodies can be used for immunoprecipitation of amplified product and detection of HCV nucleic acid microparticles or carrier coated with anti-HCV monoclonal antibody used to capture virus or viral protein associated with HCV RNA. Then detection methodology for RNA may be used.

These monoclonal antibodies also can be used for localization of HCV antigens within the cell using HCV monoclonal antibody tagged directly (fluorescence, colloidal gold, etc.) or using secondary tagged anti-mouse antibody. Histopathology of disease may be tracked. Further, the detection of native or recombinant HCV antigens in sera, tissue, cells, culture media, or body fluid using individual monoclonal antibodies in a sandwich configuration or a cocktail of monoclonal antibodies on the solid phase and in the detection system.

One step antigen assays using monoclonal antibodies against non overlapping epitopes may also be performed. Some monoclonal antibodies may recognize antigenic epitopes not recognized by the infected individual and therefore may be possible to recognize serum Ag both free and bound with human antibody. Furthermore, "cryptic" or hidden antigens or antigenic determinants may be uncovered by treatment of specimen with detergent or reducing agent or both. For example, NS5 antigen may exist in a capsid form covered by the virus envelope. Stripping the envelope with detergent should expose NS5 antigen. Monoclonal antibodies may also offer pragmatic advantages over high titer polyclonal antibody in giving greater sensitivity in assay or allowing shorter incubation times.

Further, antibody immunoassays, one or two step competitive assays, in which anti-HCV competed with labeled anti-HCV monoclonal antibody for binding to a limited number of antigenic sites. A more sensitive competitive assay may be developed in which human anti-HCV binds to HCV antigen in solution blocking or inhibiting the HCV antigen binding in HCV Antigen sandwich assay. Competitive assays using monoclonal antibodies allow a more precise mapping of human antibody epitopes and may be useful for determining virus neutralizing antibody epitopes. Some monoclonal antibodies may have virus neutralizing activity. Finally, monoclonal antibodies should be useful in immunoaffinity purification of native viral and recombinant HCV antigens and proteins.

Other variations of applications of the use of these unique monoclonal antibodies provided herein include the detection of HCV in immune complexes, and/or association with viral nucleic acid for detection of the nucleic acid by PCR, LCR, or by direct hybridization.

## Claims (Claims for the following Contracting State(s): AT, BE, CH, LI, DE, DK, FR, GB, GR, IT, NL, SE)

1. A monoclonal antibody or fragment thereof which specifically binds to HCV NS5 antigen wherein said monoclonal antibody is the monoclonal antibody secreted by hybridoma cell line ATCC HB 10855.

2. A monoclonal antibody or fragment thereof which specifically binds to HCV NS5 antigen wherein said monoclonal antibody is the monoclonal antibody secreted by hybridoma cell line ATCC HB 10854.

3. A monoclonal antibody secreted by A.T.C.C. deposit No. HB 10855.

4. A monoclonal antibody secreted by A.T.C.C. deposit No. HB 10854.

5. A hybridoma cell line that secretes a monoclonal antibody which specifically binds to HCV NS5 antigen wherein said hybridoma cell line is A.T.C.C. deposit No. HB 10855.

6. A hybridoma cell line that secretes a monoclonal antibody which specifically binds to HCV NS5 antigen wherein said hybridoma cell line is A.T.C.C. deposit No. HB 10854.

7. A sandwich assay method for determining the presence of HCV in a test sample which may contain HCV, comprising:
a. contacting the test sample with at least one anti-HCV NS5 antibody attached to a solid phase which antibody specifically binds to HCV NS5 antigen, to form a mixture, said antibody being a monoclonal antibody, or fragment thereof, or a combination of monoclonal antibodies specifically binding to HCV NS5 antigen, said monoclonal antibodies having been secreted by the hybridoma A.T.C.C. deposit No. HB 10855 or A.T.C.C. deposit No. HB 10554;
b. incubating said mixture for a time and under conditions sufficient to form antigen/antibody complexes;
c. contacting said complexes with an indicator reagent comprising a signal generating compound capable of generating a measurable detectable signal attached to an anti-HCV NS5 antibody, to form a second mixture, said antibody being a monoclonal antibody, or fragment thereof, or a combination of monoclonal antibodies specifically binding to HCV NS5 antigen, said monoclonal antibodies having been secreted by the hybridoma A.T.C.C. deposit No. HB 10855 or A.T.C.C. deposit No. HB 10854;
d. incubating said second mixture for a time and under conditions sufficient to form antibody/antigen complexes; and
e. determining the presence of HCV in the test sample by detecting the measuring signal generated, wherein the amount of HCV present in the test sample is proportional to said measurable signal.

8. The method of claim 7 wherein the signal generating compound is selected from the group consisting of a luminescent compound, a chemiluminescent compound, an enzyme and a radioactive element.

9. A sandwich assay method for determining the presence and amount of HCV which may be present in a test sample, comprising:
a. contacting a test sample with a monoclonal anti-HCV NS5 antibody attached to a solid phase and an indicator reagent comprising a monoclonal antibody which specifically binds to HCV NS5 attached to a signal generating compound, to form a mixture, said antibodies being monoclonal antibodies, or fragment thereof, or a combination of monoclonal antibodies specifically binding to HCV NS5 antigen, said monoclonal antibodies having been secreted by the hybridoma A.T.C.C. deposit No. HB 10855 or A.T.C.C. deposit No. HB 10854;
b. incubating said mixture for a time and under conditions sufficient to form antibody/antigen/antibody complexes;
c. determining the presence of HCV present in the test sample by detecting the measurable signal as an indication of the presence of HCV in the test sample, wherein the amount of HCV present in the test sample is proportional to the measurable signal generated.

10. A competitive assay method for determining the presence and amount of HCV antibody which may be present in a test sample, comprising:
a. contacting a test sample suspected of containing HCV antibodies with both a solid phase coated with HCV NS5 proteins and an indicator reagent comprising a signal generating compound and a monoclonal antibody which specifically binds to HCV NS5 proteins, for a time and under conditions sufficient to form antigen/antibody complexes of the test sample and solid phase and/or indicator reagent and solid phase, said monoclonal antibody, or fragment thereof, or a combination of said monoclonal antibodies which specifically binds to HCV NS5 proteins, said monoclonal antibody having been secreted by the hybridoma A.T.C.C. deposit No. HB 10855 or A.T.C.C. deposit No. HB 10854;
b. determining the presence of HCV antibody present in the test sample by detecting the reduction in binding of the indicator reagent to the solid phase as compared to the signal generated from a negative test sample to indicate the presence of HCV antibody in the test sample.

11. An assay kit for determining the presence of HCV antigen or antibody in a test sample comprising:
a container containing at least one monoclonal antibody, or fragment thereof, which specifically binds to HCV NS5 antigen wherein said monoclonal antibody is the monoclonal antibody secreted by the cell line A.T.C.C. deposit No. HB 10855 or A.T.C.C. deposit No. HB 10854.

## Claims (Claims for the following Contracting State(s): ES)

1. A sandwich assay method for determining the presence of HCV in a test sample which may contain HCV, comprising:
a. contacting the test sample with at least one anti-HCV NS5 antibody attached to a solid phase which antibody specifically binds to HCV NS5 antigen, to form a mixture, said antibody being a monoclonal antibody, or fragment thereof, or a combination of monoclonal antibodies specifically binding to HCV NS5 antigen, said monoclonal antibodies having been secreted by the hybridoma A.T.C.C. deposit No. HB 10855 or A.T.C.C. deposit No. HB 10854;
b. incubating said mixture for a time and under conditions sufficient to form antigen/antibody complexes;
c. contacting said complexes with an indicator reagent comprising a signal generating compound capable of generating a measurable detectable signal attached to an anti-HCV NS5 antibody, to form a second mixture, said antibody being a monoclonal antibody, or fragment thereof, or a combination of monoclonal antibodies specifically binding to HCV NS5 antigen, said monoclonal antibodies having been secreted by the hybridoma A.T.C.C. deposit No. HB 10855 or A.T.C.C. deposit No. HB 10854;
d. incubating said second mixture for a time and under conditions sufficient to form antibody/antigen complexes; and
e. determining the presence of HCV in the test sample by detecting the measurable signal generated, wherein the amount of HCV present in the test sample is proportional to said measurable signal.

2. The method of claim 1 wherein the signal generating compound is selected from the group consisting of a luminescent compound, a chemiluminescent compound, an enzyme and a radioactive element.

3. A sandwich assay method for determining the presence and amount of HCV which may be present in a test sample, comprising:
a. contacting a test sample with a monoclonal anti-HCV NS5 antibody attached to a solid phase and an indicator reagent comprising a monoclonal antibody which specifically binds to HCV NS5 attached to a signal generating compound, to form a mixture, said antibodies being monoclonal antibodies, or fragment thereof, or a combination of monoclonal antibodies specifically binding to HCV NS5 antigen, said monoclonal antibodies having been secreted by the hybridoma A.T.C.C. deposit No. HB 10855 or A.T.C.C. deposit No. HB 10854;
b. incubating said mixture for a time and under conditions sufficient to form antibody/antigen/antibody complexes;
c. determining the presence of HCV present in the test sample by detecting the measurable signal as an indication of the presence of HCV in the test sample, wherein the amount of HCV present in the test sample is proportional to the measurable signal generated.

4. A competitive assay method for determining the presence and amount of HCV antibody which may be present in a test sample, comprising:
a. contacting a test sample suspected of containing HCV antibodies with both a solid phase coated with HCV NS5 proteins and an indicator reagent comprising a signal generating compound and a monoclonal antibody which specifically binds to HCV NS5 proteins, for a time and under conditions sufficient to form antigen/antibody complexes of the test sample and solid phase and/or indicator reagent and solid phase, said monoclonal antibody, or fragment thereof, or a combination of said monoclonal antibodies which specifically binds to HCV NS5 proteins, said monoclonal antibody having been secreted by the hybridoma A.T.C.C. deposit No. HB 10855 or A.T.C.C. deposit No. HB 10854;
b. determining the presence of HCV antibody present in the test sample by detecting the reduction in binding of the indicator reagent to the solid phase as compared to the signal generated from a negative test sample to indicate the presence of HCV antibody in the test sample.

5. A process for producing a monoclonal antibody or fragment thereof which specifically binds to HCV NS5 antigen, said process comprising propagating a hybridoma cell line A.T.C.C. deposit No. HB 10855 or A.T.C.C. deposit No. HB 10854 in a medium suitable for the expression of the respective monoclonal antibodies by said cell line and purifying the expressed monoclonal antibodies.

## Patentansprüche (Patentansprüche für folgende(n) Vertragsstaat(en): AT, BE, CH, LI, DE, DK, FR, GB, GR, IT, NL, SE)

1. Ein monoklonaler Antikörper oder Fragment davon, der spezifisch an das HCV-NS5-Antigen bindet, wobei es sich bei dem monoklonalen Antikörper um den monoklonalen Antikörper handelt, der von der Hybridomzellinie ATCC HB 10855 abgesondert wird.

2. Ein monoklonaler Antikörper oder Fragment davon, der spezifisch an das HCV-NS5-Antigen bindet, wobei es sich bei dem monoklonalen Antikörper um den monoklonalen Antikörper handelt, der von der Hybridomzellinie ATCC HB 10854 abgesondert wird.

3. Ein monoklonaler Antikörper, der von A.T.C.C. Hinterlegungsnummer HB 10855 abgesondert wird.

4. Ein monoklonaler Antikörper, der von A.T.C.C. Hinterlegungsnummer HB 10854 abgesondert wird.

5. Eine Hybridomzellinie, die einen monoklonalen Antikörper absondert, der spezifisch an das HCV-NS5-Antigen bindet, wobei die Hybridomzellinie die mit der A.T.C.C. Hinterlegungsnummer HB 10855 ist.

6. Eine Hybridomzellinie, die einen monoklonalen Antikörper absondert, der spezifisch an das HCV-NS5-Antigen bindet, wobei die Hybridomzellinie die mit der A.T.C.C. Hinterlegungsnummer HB 10854 ist.

7. Ein Sandwichassayverfahren zur Bestimmung der Anwesenheit von HCV in einer Testprobe, die möglicherweise HCV enthält, das folgendes umfaßt:
a. In-Kontakt-Bringen der Testprobe mit mindestens einem anti-HCV-NS5-Antikörper, der an eine feste Phase gebunden ist und der spezifisch an das HCV-NS5-Antigen bindet, unter Ausbildung einer Mischung, wobei der Antikörper ein monoklonaler Antikörper ist oder ein Fragment davon, oder eine Kombination von monoklonalen Antikörpern, die spezifisch an das HCV-NS5-Antigen binden, wobei der monoklonale Antikörper von dem Hybridom mit der A.T.C.C. Hinterlegungsnummer HB 10855 oder der A.T.C.C. Hinterlegungsnummer HB 10854 abgesondert wurde;
b. Inkubation der Mischung eine Zeit lang und unter Bedingungen, die zur Ausbildung von Antigen/Antikörper-Komplexen hinreichend sind;
c. In-Kontakt-Bringen der Komplexe mit einem Indikatorreagenz, das eine signalerzeugende Verbindung umfaßt, die ein meßbares nachweisbares Signal erzeugen kann, und die an einen anti-HCV-NS5-Antikörper gebunden ist, unter Ausbildung einer zweiten Mischung, wobei der Antikörper ein monoklonaler Antikörper oder ein Fragment davon ist, oder eine Kombination von monoklonalen Antikörpern, die spezifisch an das HCV-NS5-Antigen binden, wobei die monoklonalen Antikörper von dem Hybridom mit der A.T.C.C. Hinterlegungsnummer HB 10855 oder der A.T.C.C. Hinterlegungsnummer HB 10854 abgesondert wurden;
d. Inkubation der zweiten Mischung eine Zeit lang und unter Bedingungen, die zur Ausbildung von Antikörper/Antigen-Komplexen hinreichend sind; und
e. Bestimmen der Anwesenheit von HCV in der Testprobe durch den Nachweis des erzeugten meßbaren Signals, wobei die Menge an in der Testprobe vorhandenem HCV proportional zum meßbaren Signal ist.

8. Das Verfahren nach Anspruch 7, worin die signalerzeugende Verbindung aus der Gruppe bestehend aus einer lumineszenten Verbindung, einer chemilumineszenten Verbindung, einem Enzym und einem radioaktiven Element gewählt ist.

9. Ein Sandwichassayverfahren zur Bestimmung der Anwesenheit und Menge von HCV, die in einer Testprobe möglicherweise vorhanden ist, das folgendes umfaßt:
a. In-Kontakt-Bringen einer Testprobe mit einem monoklonalen anti-HCV-NS5-Antikörper, der an eine feste Phase gebunden ist, und mit einem Indikatorreagenz, das einen monoklonalen Antikörper umfaßt, der spezifisch an HCV NS5 bindet und an eine signalerzeugende Verbindung gebunden ist, unter Ausbildung einer Mischung, wobei die Antikörper monoklonale Antikörper oder Fragmente davon oder eine Kombination von monoklonalen Antikörpern sind, die spezifisch an das HCV-NS5-Antigen binden, wobei die monoklonalen Antikörper von dem Hybridom mit der A.T.C.C. Hinterlegungsnummer HB 10855 oder der A.T.C.C. Hinterlegungsnummer HB 10854 abgesondert wurden;
b. Inkubation der Mischung eine Zeit lang und unter Bedingungen, die zur Ausbildung von Antikörper/Antigen/Antikörper-Komplexen hinreichend sind;
c. Bestimmen der Anwesenheit von in der Testprobe vorhandenem HCV durch den Nachweis des meßbaren Signals als Anzeige für die Anwesenheit von HCV in der Testprobe, wobei die Menge an HCV in der Testprobe proportional zum erzeugten meßbaren Signal ist.

10. Ein Kompetitivassayverfahren zum Bestimmen der Anwesenheit und Menge von HCV-Antikörper, der möglicherweise in der Testprobe vorhanden ist, das folgendes umfaßt:
a. In-Kontakt-Bringen einer Testprobe, von der vermutet wird, daß sie HCV-Antikörper enthält, mit einer festen Phase, die mit HCV-NS5-Proteinen beschichtet ist, sowie mit einem Indikatorreagenz, das eine signalerzeugende Verbindung und einen monoklonalen Antikörper umfaßt, der spezifisch an HCV-NS5-Proteine bindet, eine Zeit lang und unter Bedingungen, die zur Ausbildung von Antigen/Antikörper-Komplexen aus der Testprobe und der festen Phase und/oder dem Indikatorreagenz und der festen Phase hinreichend sind, wobei der monoklonale Antikörper oder das Fragment davon oder eine Kombination von monoklonalen Antikörpern, die spezifisch an HCV-NS5-Proteine binden, von dem Hybridom mit der A.T.C.C. Hinterlegungsnummer HB 10855 oder der A.T.C.C. Hinterlegungsnummer HB 10854 abgesondert wurden;
b. Bestimmen der Anwesenheit von in der Testprobe vorhandenem HCV-Antikörper durch Nachweis des Rückgangs der Bindung des Indikatorreagenzes an die feste Phase verglichen mit dem Signal, das von einer negativen Testprobe erzeugt wird, um die Anwesenheit von HCV-Antikörper in der Testprobe anzuzeigen.

11. Ein Assaykit zur Bestimmung der Anwesenheit von HCV-Antigen oder -Antikörper in einer Testprobe, das folgendes umfaßt:
einen Behälter, der wenigstens einen monoklonalen Antikörper oder ein Fragment davon enthält, der/das spezifisch an das HCV-NS5-Antigen bindet, wobei es sich bei dem monoklonalen Antikörper um den monoklonalen Antikörper handelt, der von der Hybridomzellinie mit der A.T.C.C. Hinterlegungsnummer HB 10855 oder der A.T.C.C. Hinterlegungsnummer HB 10854 abgesondert wird.

## Patentansprüche (Patentansprüche für folgende(n) Vertragsstaat(en): ES)

1. Ein Sandwichassayverfahren zur Bestimmung der Anwesenheit von HCV in einer Testprobe, die möglicherweise HCV enthält, das folgendes umfaßt:
a. In-Kontakt-Bringen der Testprobe mit mindestens einem anti-HCV-NS5-Antikörper, der an eine feste Phase gebunden ist und der spezifisch an das HCV-NS5-Antigen bindet, unter Ausbildung einer Mischung, wobei der Antikörper ein monoklonaler Antikörper ist oder ein Fragment davon, oder eine Kombination von monoklonalen Antikörpern, die spezifisch an das HCV-NS5-Antigen binden, wobei der monoklonale Antikörper von dem Hybridom mit der A.T.C.C. Hinterlegungsnummer HB 10855 oder der A.T.C.C. Hinterlegungsnummer HB 10854 abgesondert wurde;
b. Inkubation der Mischung eine Zeit lang und unter Bedingungen, die zur Ausbildung von Antigen/Antikörper-Komplexen hinreichend sind;
c. In-Kontakt-Bringen der Komplexe mit einem Indikatorreagenz, das eine signalerzeugende Verbindung umfaßt, die ein meßbares nachweisbares Signal erzeugen kann, und die an einen anti-HCV-NS5-Antikörper gebunden ist, unter Ausbildung einer zweiten Mischung, wobei der Antikörper ein monoklonaler Antikörper oder ein Fragment davon ist, oder eine Kombination von monoklonalen Antikörpern, die spezifisch an das HCV-NS5-Antigen binden, wobei die monoklonalen Antikörper von dem Hybridom mit der A.T.C.C. Hinterlegungsnummer HB 10855 oder der A.T.C.C. Hinterlegungsnummer HB 10854 abgesondert wurden;
d. Inkubation der zweiten Mischung eine Zeit lang und unter Bedingungen, die zur Ausbildung von Antikörper/Antigen-Komplexen hinreichend sind; und
e. Bestimmen der Anwesenheit von HCV in der Testprobe durch den Nachweis des erzeugten meßbaren Signals, wobei die Menge an in der Testprobe vorhandenem HCV proportional zum meßbaren Signal ist.

2. Das Verfahren nach Anspruch 1, worin die signalerzeugende Verbindung aus der Gruppe bestehend aus einer lumineszenten Verbindung, einer chemilumineszenten Verbindung, einem Enzym und einem radioaktiven Element gewählt ist.

3. Ein Sandwichassayverfahren zur Bestimmung der Anwesenheit und Menge von HCV, die in einer Testprobe möglicherweise vorhanden ist, das folgendes umfaßt:
a. In-Kontakt-Bringen einer Testprobe mit einem monoklonalen anti-HCV-NS5-Antikörper, der an eine feste Phase gebunden ist, und mit einem Indikatorreagenz, das einen monoklonalen Antikörper enthält, der spezifisch an HCV NS5 bindet und an eine signalerzeugende Verbindung gebunden ist, unter Ausbildung einer Mischung, wobei die Antikörper monoklonale Antikörper oder Fragmente davon oder eine Kombination von monoklonalen Antikörpern sind, die spezifisch an das HCV-NS5-Antigen binden, wobei die monoklonalen Antikörper von dem Hybridom mit der A.T.C.C. Hinterlegungsnummer HB 10855 oder der A.T.C.C. Hinterlegungsnummer HB 10854 abgesondert wurden;
b. Inkubation der Mischung eine Zeit lang und unter Bedingungen, die zur Ausbildung von Antikörper/Antigen/Antikörper-Komplexen hinreichend sind;
c. Bestimmen der Anwesenheit von in der Testprobe vorhandenem HCV durch den Nachweis des meßbaren Signals als Anzeige für die Anwesenheit von HCV in der Testprobe, wobei die Menge an in der Testprobe vorhandenem HCV proportional zum erzeugten meßbaren Signal ist.

4. Ein Kompetitivassayverfahren zum Bestimmen der Anwesenheit und Menge von HCV-Antikörper, der möglicherweise in der Testprobe vorhanden ist, das folgendes umfaßt:
a. In-Kontakt-Bringen einer Testprobe, von der vermutet wird, daß sie HCV-Antikörper enthält, mit einer festen Phase, die mit HCV-NS5-Proteinen beschichtet ist, sowie mit einem Indikatorreagenz, das eine signalerzeugende Verbindung und einen monoklonalen Antikörper umfaßt, der spezifisch an HCV-NS5-Proteine bindet, eine Zeit lang und unter Bedingungen, die zur Ausbildung von Antigen/Antikörper-Komplexen aus der Testprobe und der festen Phase und/oder dem Indikatorreagenz und der festen Phase hinreichend sind, wobei der monoklonale Antikörper oder das Fragment davon oder eine Kombination von monoklonalen Antikörpern, die spezifisch an HCV-NS5-Proteine binden, von dem Hybridom mit der A.T.C.C. Hinterlegungsnummer HB 10855 oder der A.T.C.C. Hinterlegungsnummer HB 10854 abgesondert wurden;
b. Bestimmen der Anwesenheit von in der Testprobe vorhandenem HCV-Antikörper durch Nachweis des Rückgangs der Bindung des Indikatorreagenzes an die feste Phase verglichen mit dem Signal, das von einer negativen Testprobe erzeugt wird, um die Anwesenheit von HCV-Antikörper in der Testprobe anzuzeigen.

5. Ein Verfahren für die Herstellung eines monoklonalen Antikörpers oder eines Fragmentes davon, der/das spezifisch an das HCV-NS5-Antigen bindet, wobei das Verfahren die Vermehrung einer Hybridomzellinie mit der A.T.C.C. Hinterlegungsnummer HB 10855 oder der A.T.C.C. Hinterlegungsnummer HB 10854 in einem Medium, das für die Expression der jeweiligen monoklonalen Antikörper durch diese Zellinie geeignet ist, und die Reinigung der exprimierten monoklonalen Antikörper umfaßt.

## Revendications (Revendications pour l'(les) Etat(s) contractant(s) suivant(s): AT, BE, CH, LI, DE, DK, FR, GB, GR, IT, NL, SE)

1. Anticorps monoclonal ou fragment de celui-ci qui se lie spécifiquement à l'antigène NS5 de HCV où ledit anticorps monoclonal est l'anticorps monoclonal sécrété par la lignée cellulaire d'hybridome ATCC HB 10855.

2. Anticorps monoclonal ou fragment de celui-ci qui se lie spécifiquement à l'antigène NS5 de HCV où ledit anticorps monoclonal est l'anticorps monoclonal sécrété par la lignée cellulaire d'hybridomes ATCC HB 10854.

3. Anticorps monoclonal sécrété par le dépôt ATCC n° HB 10855.

4. Anticorps monoclonal sécrété par le dépôt ATCC n° HB 10854.

5. Lignée cellulaire d'hybridomes qui sécrète un anticorps monoclonal qui se lie spécifiquement à l'antigène NS5 de HCV où ladite lignée cellulaire d'hybridomes est le dépôt ATCC n° HB 10855.

6. Lignée cellulaire d'hybridomes qui sécrète un anticorps monoclonal qui se lie spécifiquement à l'antigène NS5 de HCV où ladite lignée cellulaire d'hybridomes est le dépôt ATCC n° HB 10854.

7. Procédé de dosage sandwich pour déterminer la présence de HCV dans un échantillon test qui peut contenir HCV, comprenant :
a. la mise en contact de l'échantillon test avec au moins un anticorps anti-NS5 de HCV fixé à une phase solide, lequel anticorps se lie spécifiquement à l'antigène NS5 de HCV, pour former un mélange, ledit anticorps étant un anticorps monoclonal, ou un fragment de celui-ci, ou une combinaison d'anticorps monoclonaux qui se lient spécifiquement à l'antigène NS5 de HCV, lesdits anticorps monoclonaux ayant été sécrétés par l'hybridome dépôt ATCC n° HB 10855 ou dépôt ATCC n° HB 10854 ;
b. l'incubation dudit mélange pendant une durée et dans des conditions suffisantes pour former des complexes antigène/anticorps;
c. la mise en contact desdits complexes avec un réactif indicateur comprenant un composé générateur de signal capable de générer un signal détectable mesurable fixé à un anticorps anti-NS5 de HCV, pour former un second mélange, ledit anticorps étant un anticorps monoclonal, ou un fragment de celui-ci, ou une combinaison d'anticorps monoclonaux qui se lient spécifiquement à l'antigène NS5 de HCV, lesdits anticorps monoclonaux ayant été sécrétés par l'hybridome dépôt ATCC n° HB 10855 ou dépôt ATCC n° HB 10854 ;
d. l'incubation dudit second mélange pendant une durée et dans des conditions suffisantes pour former des complexes anticorps/antigène ; et
e. la détermination de la présence de HCV dans l'échantillon test par détection du signal mesurable généré, où la quantité de HCV présente dans l'échantillon test est proportionnelle audit signal mesurable.

8. Procédé selon la revendication 7 dans lequel le composé générateur de signal est choisi dans le groupe consistant en un composé luminescent, un composé chimiluminescent, une enzyme et un élément radioactif.

9. Procédé de dosage sandwich pour déterminer la présence et la quantité de HCV qui peut être présent dans un échantillon test, comprenant :
a. la mise en contact d'un échantillon test avec un anticorps monoclonal anti-NS5 de HCV fixé à une phase solide et un réactif indicateur comprenant un anticorps monoclonal qui se lie spécifiquement à NS5 de HCV fixé à un composé générateur de signal, pour former un mélange, lesdits anticorps étant des anticorps monoclonaux, ou un fragment de ceux-ci, ou une combinaison d'anticorps monoclonaux qui se lient spécifiquement à l'antigène NS5 de HCV, lesdits anticorps monoclonaux ayant été sécrétés par l'hybridome dépôt ATCC n° HB 10855 ou ATCC n° HB 10854 ;
b. l'incubation dudit mélange pendant une durée et dans des conditions suffisantes pour former des complexes anticorps/antigène/anticorps ;
c. la détermination de la présence de HCV présent dans l'échantillon test par détection du signal mesurable en tant qu'indication de la présence de HCV dans l'échantillon test, où la quantité de HCV présente dans l'échantillon test est proportionnelle au signal mesurable généré.

10. Procédé de dosage compétitif pour déterminer la présence et la quantité d'un anticorps de HCV qui peut être présent dans un échantillon test, comprenant:
a. la mise en contact d'un échantillon test supposé contenir des anticorps de HCV avec une phase solide revêtue de protéines NS5 de HCV et un réactif indicateur comprenant un composé générateur de signal et un anticorps monoclonal qui se lie spécifiquement à des protéines NS5 de HCV, pendant une durée et dans des conditions suffisantes pour former des complexes antigène/anticorps de l'échantillon test et de la phase solide et/ou du réactif indicateur et de la phase solide, dudit anticorps monoclonal, ou d'un fragment de celui-ci, ou d'une combinaison desdits anticorps monoclonaux qui se lient spécifiquement à des protéines NS5 de HCV, ledit anticorps monoclonal ayant été sécrété par l'hybridome dépôt ATCC n° HB 10855 ou dépôt ATCC n° HB 10854 ;
b. la détermination de la présence d'un anticorps de HCV présent dans l'échantillon test par détection de la réduction de la liaison du réactif indicateur à la phase solide par rapport au signal généré par un échantillon test négatif pour indiquer la présence d'un anticorps de HCV dans l'échantillon test.

11. Trousse de dosage pour déterminer la présence d'un antigène ou anticorps de HCV dans un échantillon test comprenant :
un récipient contenant au moins un anticorps monoclonal, ou un fragment de celui-ci, qui se lie spécifiquement à l'antigène NS5 de HCV, où ledit anticorps monoclonal est l'anticorps monoclonal sécrété par la lignée cellulaire dépôt ATCC n° HB 10855 ou dépôt ATCC n° HB 10854.

## Revendications (Revendications pour l'(les) Etat(s) contractant(s) suivant(s): ES)

1. Procédé de dosage sandwich pour déterminer la présence de HCV dans un échantillon test qui peut contenir HCV, comprenant :
a. la mise en contact de l'échantillon test avec au moins un anticorps anti-NS5 de HCV fixé à une phase solide, lequel anticorps se lie spécifiquement à l'antigène NS5 de HCV, pour former un mélange, ledit anticorps étant un anticorps monoclonal, ou un fragment de celui-ci, ou une combinaison d'anticorps monoclonaux qui se lient spécifiquement à l'antigène NS5 de HCV, lesdits anticorps monoclonaux ayant été sécrétés par l'hybridome dépôt ATCC n° HB 10855 ou dépôt ATCC n° HB 10854 ;
b. l'incubation dudit mélange pendant une durée et dans des conditions suffisantes pour former des complexes antigène/anticorps;
c. la mise en contact desdits complexes avec un réactif indicateur comprenant un composé générateur de signal capable de générer un signal détectable mesurable fixé à un anticorps anti-NS5 de HCV, pour former un second mélange, ledit anticorps étant un anticorps monoclonal, ou un fragment de celui-ci, ou une combinaison d'anticorps monoclonaux qui se lient spécifiquement à l'antigène NS5 de HCV, lesdits anticorps monoclonaux ayant été sécrétés par l'hybridome dépôt ATCC n° HB 10855 ou dépôt ATCC n° HB 10854 ;
d. l'incubation dudit second mélange pendant une durée et dans des conditions suffisantes pour former des complexes anticorps/antigène ; et
e. la détermination de la présence de HCV dans l'échantillon test par détection du signal mesurable généré, où la quantité de HCV présente dans l'échantillon test est proportionnelle audit signal mesurable.

2. Procédé selon la revendication 1 dans lequel le composé générateur de signal est choisi dans le groupe consistant en un composé luminescent, un composé chimiluminescent, une enzyme et un élément radioactif.

3. Procédé de dosage sandwich pour déterminer la présence et la quantité de HCV qui peut être présent dans un échantillon test, comprenant :
a. la mise en contact d'un échantillon test avec un anticorps monoclonal anti-NS5 de HCV fixé à une phase solide et un réactif indicateur comprenant un anticorps monoclonal qui se lie spécifiquement à NS5 de HCV fixé à un composé générateur de signal, pour former un mélange, lesdits anticorps étant des anticorps monoclonaux, ou un fragment de ceux-ci, ou une combinaison d'anticorps monoclonaux qui se lient spécifiquement à l'antigène NS5 de HCV, lesdits anticorps monoclonaux ayant été sécrétés par l'hybridome dépôt ATCC n° HB 10855 ou ATCC n° HB 10854 ;
b. l'incubation dudit mélange pendant une durée et dans des conditions suffisantes pour former des complexes anticorps/antigène/anticorps ;
c. la détermination de la présence de HCV présent dans l'échantillon test par détection du signal mesurable en tant qu'indication de la présence de HCV dans l'échantillon test, où la quantité de HCV présente dans l'échantillon test est proportionnelle au signal mesurable généré.

4. Procédé de dosage compétitif pour déterminer la présence et la quantité d'un anticorps de HCV qui peut être présent dans un échantillon test, comprenant:
a. la mise en contact d'un échantillon test supposé contenir des anticorps de HCV avec une phase solide revêtue de protéines NS5 de HCV et un réactif indicateur comprenant un composé générateur de signal et un anticorps monoclonal qui se lie spécifiquement à des protéines NS5 de HCV, pendant une durée et dans des conditions suffisantes pour former des complexes antigène/anticorps de l'échantillon test et de la phase solide et/ou du réactif indicateur et de la phase solide, dudit anticorps monoclonal, ou d'un fragment de celui-ci, ou d'une combinaison desdits anticorps monoclonaux se lient spécifiquement à des protéines NS5 de HCV, ledit anticorps monoclonal ayant été sécrété par l'hybridome dépôt ATCC n° HB 10855 ou dépôt ATCC n° HB 10854 ;
b. la détermination de la présence d'un anticorps de HCV présent dans l'échantillon test par détection de la réduction de la liaison du réactif indicateur à la phase solide par rapport au signal généré par un échantillon test négatif pour indiquer la présence d'un anticorps de HCV dans l'échantillon test.

5. Procédé pour produire un anticorps monoclonal ou un fragment de celui-ci qui se lie spécifiquement à l'antigène NS5 de HCV, ledit procédé comprenant la propagation d'une lignée cellulaire d'hybridomes dépôt ATCC n° HB 10855 ou dépôt ATCC n° HB 10854 dans un milieu approprié à l'expression des anticorps monoclonaux respectifs par ladite lignée cellulaire et la purification des anticorps monoclonaux exprimés.


### SEQUENCE LISTING

(1) GENERAL INFORMATION:
   (i) APPLICANT: MEHTA, S.
      JOHNSON-PAEPKE, J.
      DAILEY, S.
      DESAI, S.
      DEVARE, S.
   (ii) TITLE OF INVENTION: MONOCLONAL ANTIBODIES TO PUTATIVE HCV NS5 PROTEINS AND METHODS FOR USING SAME
   (iii) NUMBER OF SEQUENCES: 4
   (iv) CORRESPONDENCE ADDRESS:
      (A) ADDRESSEE: ABBOTT LABORATORIES
      (B) STREET: ONE ABBOTT PARK ROAD
      (C) CITY: ABBOTT PARK
      (D) STATE: ILLINOIS
      (E) COUNTRY: U.S.
      (F) ZIP: 60065-3500
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.25
   (vi) CURRENT APPUCATION DATA:
      (A) APPLICATION NUMBER:
      (B) FILING DATE:
      (C) CLASSIFICATION:
   (viii) ATTORNEY/AGENT INFORMATION:
      (A) NAME: POREMBSKI, PRISCILLA E.
      (B) REGISTRATION NUMBER: 33,207
      (C) REFERENCE/DOCKET NUMBER: 4834PC.05
   (ix) TELECOMMUNICATION INFORMATION:
      (A) TELEPHONE: 708-937-6365
      (B) TELEFAX: 708-937-9556
(2) INFORMATION FOR SEQ ID NO:1:
   (i) SEQUENCE CHARACTERISTICS:
      (A) LENGTH: 517 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:1:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Pro Trp Thr His Tyr Val Pro Glu Ser Asp
   245 250 255
   Ala Ala Ala Arg Val Thr Ala Ile Leu Ser Ser Leu Thr Val Thr Gln
   260 265 270
   Leu Leu Arg Arg Leu His Gln Trp Ile Ser Ser Glu Cys Thr Thr Pro
   275 280 285
   Cys Ser Gly Ser Trp Leu Arg Asp Ile Trp Asp Trp Ile Cys Glu Val
   290 295 300
   Leu Ser Asp Phe Lys Thr Trp Leu Lys Ala Lys Leu Met Pro Gln Leu
   305 310 315 320
   Pro Gly Ile Pro Phe Val Ser Cys Gln Arg Gly Tyr Lys Gly Val Trp
   325 330 335
   Arg Val Asp Gly Ile Met His Thr Arg Cys His Cys Gly Ala Glu Ile
   340 345 350
   Thr Gly His Val Lys Asn Gly Thr Met Arg Ile Val Gly Pro Arg Thr
   355 360 365
   Cys Arg Asn Met Trp Ser Gly Thr Phe Pro Ile Asn Ala Tyr Thr Thr
   370 375 380
   Gly Pro Cys Thr Pro Leu Pro Ala Pro Asn Tyr Thr Phe Ala Leu Trp
   385 390 395 400
   Arg Val Ser Ala Glu Glu Tyr Val Glu Ile Arg Gln Val Gly Asp Phe
   405 410 415
   His Tyr Val Thr Gly Met Thr Thr Asp Asn Leu Lys Cys Pro Cys Gln
   420 425 430
   Val Pro Ser Pro Glu Phe Phe Thr Glu Leu Asp Gly Val Arg Leu His
   435 440 445
   Arg Phe Ala Pro Pro Cys Lys Pro Leu Leu Arg Glu Glu Val Ser Phe
   450 455 460
   Arg Val Gly Leu His Glu Tyr Pro Val Gly Ser Gln Leu Pro Cys Glu
   465 470 475 480
   Pro Glu Pro Asp Val Ala Val Leu Thr Ser Met Leu Thr Asp Pro Ser
   485 490 495
   His Ile Thr Ala Glu Ala Ala Gly Arg Arg Leu Asp Pro Leu Asp Cys
   500 505 510
   Arg His Ala Lys Xaa
   515
(2) INFORMATION FOR SEQ ID NO:2
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Leu Arg Arg Leu His Gln Trp Ile Ser Ser Gly Cys Thr Thr Pro
   1 5 10 15
(2) INFORMATION FOR SEQ ID NO:3
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Gly Thr Met Arg Ile Val Gly Pro Arg Thr Cys Arg Asn Met Trp
   1 5 10 15
(2) INFORMATION FOR SEQ ID NO:4
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Pro Leu Pro Ala Pro Asn Tyr Thr Phe Ala Leu Trp Arg Val Ser Ala
   1 5 10 15
   Glu Glu Tyr Val
   20